# EUROPÄISCHE PATENTANMELDUNG

(11) **EP 4 134 053 A1**
(43) Veröffentlichungstag der Anmeldung: **15.02.2023**
(21) Anmeldenummer: 22192365.9
(22) Anmeldetag: 04.10.2019
(51) Int. Cl.: A61F 13/58, A61F 13/62

(54) **WEGWERFBARE WINDEL**

(30) Priorität: 08.10.2018 DE 202018105751 U
(62) Teilanmeldung aus: 19786743.5
(71) Anmelder: LK Mahnke GmbH & Co., KG, 45479 Mühlheim and der Ruhr (DE)
(72) Erfinder: Haas, Alexandra, 42781 Haan (DE)
(74) Vertreter: Gottschald Patentanwälte Partnerschaft mbB

(57) **Zusammenfassung**

1. Wegwerfbare Windel, insbesondere Babywindel, mit einem körperseitigen Deckblatt (1), mit einem körperfernen Rückenblatt (2) und mit mindestens einer Befestigungslasche (6), wobei das Rückenblatt (2) eine Vliesschicht (14) aufweist und wobei die Befestigungslasche (6) Hakenbereiche (12) und Kleberbereiche (13) aufweist, die im Haltezustand jeweils mit dem Rückenblatt (2) in Kontakt kommen. Es wird vorgeschlagen, dass die Vliesschicht (14) des Rückenblatts (2) in einem Bondingmuster (15) aus Musterelementen (16) gebondet ist und dass im Haltezustand innerhalb der Musterelemente (16) die Kleberbereiche (13) einen mittleren Flächenanteil von mindestens 20% bezogen auf die Gesamtfläche des jeweiligen Musterelements (16) einnehmen.

## Beschreibung

Die Erfindung betrifft eine wegwerfbare Windel, insbesondere Babywindel, nach dem Oberbegriff des Anspruchs 1.

Unter wegwerfbaren Windeln, auch als Wegwerfwindel oder Einwegwindel bezeichnet, werden körpernah eingesetzte saugfähige Produkte, insbesondere Babywindeln, aber auch Erwachsenenwindeln, Inkontinenzwindeln oder dergleichen verstanden. Diese weisen ein flüssigkeitsdurchlässiges, körperseitiges Deckblatt (Topsheet) und ein flüssigkeitsundurchlässiges, meist atmungsaktives, körperfernes Rückenblatt (Backsheet) auf, zwischen denen üblicherweise eine flüssigkeitsabsorbierende Sauganordnung mit einem Saugkörper und einer Verteilerlage angeordnet ist. Der Schichtaufbau mit Deckblatt, Sauganordnung und Rückenblatt weist seitliche Befestigungslaschen auf, die ein Trägermaterial, insbesondere eine Trägerfolie, aufweisen, auf der ein Klettmaterial bildende Hakenbereiche angeordnet sind. Zum Befestigen der Windel am Körper des Benutzers werden die Befestigungslaschen über ihre Hakenbereiche mit einer zugeordneten Befestigungsfläche (Komfortbündchen) auf dem Rückenblatt zusammengebracht, wodurch eine Klettverbindung gebildet wird. Die mit dem jeweiligen Hakenbereich zusammenwirkende Befestigungsfläche wird von einem Bereich des Rückenblatts, der mit einer Vliesschicht versehen ist, gebildet, in welchem sich der jeweilige Hakenbereich verhaken kann.

Die bekannte Windel (US 9,314,962 B2), von der die Erfindung ausgeht, weist Befestigungslaschen mit Hakenbereichen und Kleberbereichen auf, die im Haltezustand bzw. im bestimmungsgemäß am Körper des Benutzers befestigten Zustand der Windel jeweils mit der Vliesschicht des Befestigungsbereichs in Kontakt kommen. Die Hakenbereiche werden dabei von einem quer zu dessen Erstreckungsrichtung geschlitzten und zu einem Streckgitter auseinander gezogenen Hakenband gebildet, wobei die einzelnen Stege des Streckgitters jeweils mehrere Haken aufweisen. In den Gitterzwischenräumen befinden sich die zugänglichen Kleberbereiche, die von einer Kleberschicht gebildet werden, über die das den jeweiligen Hakenbereich bildende Streckgitter an der Trägerfolie der Befestigungslasche befestigt ist.

Durch die Kombination aus Hakenbereichen und Kleberbereichen, die beide jeweils haltend mit der Vliesschicht zusammenwirken, wird eine gute Verbindung erreicht, die relativ hohen Normalkräften und Querkräften standhält, wodurch die Windel sicher am Körper des Benutzers befestigbar ist. Die Verbindung zwischen Befestigungslasche und Vliesschicht des Rückenblatts ist aber noch weiter optimierungsfähig.

Der Erfindung liegt das Problem zugrunde, eine wegwerfbare Windel der eingangs genannten Art derart auszugestalten und weiterzubilden, dass bei einfachem Aufbau die Verbindung zwischen Befestigungslasche und Vliesschicht weiter verbessert wird.

Wesentlich ist die grundsätzliche Überlegung, zum einen die Vliesschicht (Nonwoven-Schicht), die zum Verhaken mit Hakenbereichen dient, in einem Muster zu bonden und zum anderen an der jeweiligen Befestigungslasche, von der insbesondere an jeder Seite der Windel jeweils eine vorgesehen ist, zusätzlich zu Hakenbereichen auch Kleberbereiche in einem bestimmten Flächenanteil, nämlich von mindestens 20%, vorzusehen, die zusammen mit den Hakenbereichen mit der jeweiligen Vliesschicht im Haltezustand in Kontakt bringbar sind. Im Haltezustand, wenn also die Windel beispielsweise am Körper des Benutzers befestigt ist, erfolgt eine Fixierung der Befestigungslasche an der Vliesschicht zum einen durch die Hakenbereiche, nämlich durch Verhaken, und zum anderen durch die Kleberbereiche, nämlich durch Verkleben.

Mit dem "Bonden", durch das das Muster erzeugt wird, ist hier ein Verfestigen der Vliesschicht durch Verprägen gemeint, was im Folgenden noch näher erläutert wird. Die Vliesschicht, die auf diese Weise verfestigt wird, kann selbst auch schon mechanisch, chemisch und/oder thermisch vorverfestigt worden sein, beispielsweise wasserstrahlverfestigt oder thermoverfestigt. Die Vliesschicht, die dem Verfahrensschritt des Bondens durch Verprägen und/oder dem Verfahrensschritt des Vorverfestigens ausgesetzt worden ist, kann wiederum von einem Vlies aus einer Polymerschmelze (Spinnvlies), von einem nassgelegten Vlies (wetlaid) oder von einem trockengelegten Vlies (drylaid), insbesondere von einem mechanisch trockengelegten Vlies (carded) oder von einem aerodynamisch trockengelegten Vlies (airlaid), gebildet worden sein.

Durch das Bonden wird die Vliesschicht an einer Vielzahl von Stellen unter Anwendung erhöhten Drucks und erhöhter Temperatur verprägt, das heißt verschweißt, wodurch eine Verfestigung der Vliesschicht erzeugt wird. Ein solches Bonden wird beispielsweise in einem Prägekalander oder in einer Ultraschall-Schweißanlage durchgeführt, um nur einige Beispiele zu nennen. An den Stellen, an denen das Verprägen bzw. Verschweißen erfolgt, werden Prägeelemente, beispielsweise Prägepunkte und/oder Prägelinien, erzeugt, die einzelne Musterelemente bilden, die zusammen wiederrum ein Bondingmuster bilden. Im Bereich der Prägeelemente ist das Fasermaterial der Vliesschicht komprimiert, wohingegen es an den übrigen Stellen ein größeres Volumen einnimmt. An diesen übrigen Stellen, die gegenüber der Ebene, entlang der das Rückenblatt verläuft, weiter hervorstehen, erfolgt dann im Haltezustand der Kontakt mit den Hakenbereichen und Kleberbereichen. Dabei ist es denkbar, die Vliesschicht allein oder zusammen mit einem Vliesschicht-Trägermaterial, beispielsweise in Form einer Folie, einem Gewebe oder einer weiteren Vliesschicht, zu bonden. Wird die Vliesschicht nicht zusammen mit einem Vliesschicht-Trägermaterial gebondet, kann die Vliesschicht zur Bildung des Rückenblatts auch nach dem Bonden mit einem Vliesschicht-Trägermaterial, beispielsweise in Form einer Folie, einem Gewebe oder einer weiteren Vliesschicht, verklebt werden. Grundsätzlich kann die Vliesschicht auch allein das Rückenblatt bilden.

Durch das Bonden werden nun mehrere Fasern im Bereich des jeweiligen Prägeelements, beispielsweise des jeweiligen Prägepunkts, miteinander und gegebenenfalls mit dem Vliesstoff-Trägermaterial verschmolzen und dadurch an dieser Stelle fixiert. Von diesen im Bereich der Prägeelemente fixierten Fasern erstrecken sich mehrere auch in den übrigen, nicht-verschweißten Abschnitt des jeweiligen Musterelements, der zum Kontakt mit den Hakenbereichen und Kleberbereichen dient. Durch den Kleber der Kleberbereiche wird dann eine Vielzahl von Fasern der Vliesschicht mit der Befestigungslasche verklebt und dadurch fixiert. Zumindest einige der Fasern sind dann mit einem Abschnitt an der jeweiligen Prägestelle und mit einem anderen Abschnitt an der jeweiligen Stelle der Verklebung mit dem Kleberbereich fixiert. Diese zumindest zweifach fixierten Fasern können vergleichsweise hohe Haltekräfte aufnehmen. Verhaken sich also Haken der Hakenbereiche an diesen zumindest zweifach fixierten Fasern, die eine Halteschlaufe bilden können, wird dadurch eine besonders feste Verbindung zwischen Befestigungslasche und Rückenblatt erzeugt. Dabei hat sich herausgestellt, dass ein mittlerer Flächenanteil von mindestens 20% der Kleberbereiche bezogen auf die Gesamtfläche des jeweiligen Musterelements gewährleistet, das besonders viele der Fasern durch den Kleber fest fixiert werden können. Indem nun die Vliesschicht auch in einem Bondingmuster aus Musterelementen gebondet ist, werden die Fasern auch an einer Vielzahl von Stellen regelmäßig im Rückenblatt fixiert. Zusammen ergibt sich dadurch eine besonders hohe Festigkeit einer Verbindung zwischen Befestigungslasche und Vliesschicht im Haltezustand, die nicht nur relativ hohen Normalkräften, also Kräften orthogonal zur Verlaufsebene des Rückenblatts, sondern gerade auch relativ hohen Scherkräften, also Querkräften entlang dieser Verlaufsebene, standhält. So weist die Verbindung zwischen Befestigungslasche und Vliesschicht im Haltezustand insbesondere eine absolute Scherfestigkeit von mindestens 10 N, vorzugsweise von mindestens 15 N, weiter vorzugsweise von mindestens 20 N, auf. Die Scherfestigkeit wird dabei nach SGS Courtray Methode C52b bestimmt.

Dies wiederrum führt zu einem besonders sicheren Sitz der Windel am Körper des Benutzers. Die vorschlagsgemäße Lösung hat auch den Vorteil, dass auf eine Befestigungsfläche, die von einem separaten Komfortbündchen gebildet wird, verzichtet werden kann, was den Aufbau der Windel vereinfacht. Dies führt auch zu einer Materialreduzierung und ist somit auch unter Nachhaltigkeitsgesichtspunkten vorteilhaft.

Im Einzelnen wird vorgeschlagen, dass die Vliesschicht des Rückenblatts in einem Bondingmuster aus Musterelementen gebondet ist und dass im Haltezustand innerhalb der Musterelemente die Kleberbereiche einen mittleren Flächenanteil von mindestens 20% bezogen auf die Gesamtfläche des jeweiligen Musterelements einnehmen. Es nimmt also bei denjenigen Musterelementen, die im Haltezustand mit einem der Hakenbereiche in Kontakt kommen, bei einer vertikalen Projektion auf die übereinander angeordneten Lagen aus Vliesschicht und Befestigungslasche die Projektionsfläche der Kleberbereiche mindestens 20% ein. Dies gilt jedenfalls für eine Ausrichtung der Befestigungslasche im Haltezustand parallel zur Vorderkante des Rückenblatts, also der das vordere Ende der Windel definierenden, entlang der Taille des Benutzers verlaufenden Kante. Der übrige Flächenanteil innerhalb des jeweiligen Musterelements steht dann für ein Verhaken mit den Hakenbereichen zur Verfügung. "Mittlerer Flächenanteil" meint dabei, dass die Kleberbereiche bei jedem Musterelement, das im Haltezustand mit einem der Hakenbereiche in Kontakt kommt, zwar einen anderen Flächenanteil bezogen auf die Gesamtfläche des jeweiligen Musterelements einnehmen können, der Flächenanteil der Kleberbereiche aber bezogen auf alle Musterelemente, die im Haltezustand mit einem der Hakenbereiche in Kontakt kommen, durchschnittlich mindestens 20% beträgt.

Die Ansprüche 2 und 3 definieren weitere bevorzugte Wertebereiche für den mittleren Flächenanteil der Kleberbereiche und für den mittleren Flächenanteil der Hakenbereiche. Die Kleberbereiche, jedenfalls einige und insbesondere die meisten davon, sind dabei zwischen Hakenbereichen angeordnet und/oder von Hakenbereichen vollständig umgeben. Entsprechende Hakenbereiche mit Zwischenräumen zur Ausbildung der Kleberbereiche werden vorzugsweise durch ein eine Vielzahl von Haken aufweisendes, mehrfach geschlitztes Band gebildet, das quer zum Verlauf der Schlitze auseinander gezogen bzw. gestreckt ist und dadurch ein Streckgitter bildet. Das Streckgitter weist dann eine Vielzahl von miteinander in regelmäßigen Abständen verbundenen, mit Haken versehenen Stegen auf, die die Hakenbereiche bilden. Wird ein solches mit Haken versehenes Band als Streckgitter, also im gestreckten Zustand, auf eine vorzugsweise durchgehende Kleberschicht aufgesetzt, werden einzelne Bereiche der Kleberschicht nicht von den Hakenbereichen abgedeckt. Dies sind dann die Kleberbereiche. Dabei können nicht nur Kleberbereiche innerhalb der Hakenbereiche, sondern auch außerhalb des die Haken aufweisenden Hakenbandes, insbesondere zu seinen beiden Seiten je ein Kleberbereich, vorgesehen sein.

Nach der bevorzugten Ausgestaltung gemäß Anspruch 4 nehmen die Musterelemente, die durch das Bonden erzeugt worden sind, zumindest den größten Teil der Fläche der Vliesschicht und vorzugsweise die gesamte Fläche der Vliesschicht ein. Zusätzlich oder alternativ nimmt die Vliesschicht zumindest den größten Teil der Fläche des Rückenblatts und insbesondere die gesamte Fläche des Rückenblatts ein. Besonders bevorzugt ist also das gesamte Rückenblatt mit dem Bondingmuster versehen, was es besonders einfach macht, die jeweilige Befestigungslasche mit der Vliesschicht zu verbinden.

Die Musterelemente, die durch das Bonden erzeugt werden, sind vorzugsweise ohne Zwischenräume angeordnet und/oder haben alle denselben Querschnitt, das heißt dieselbe Querschnittsform und -fläche (Anspruch 5). "Ohne Zwischenräume" meint, dass die Begrenzung des einen Musterelements an einer Seite auch gleichzeitig die Begrenzung des benachbarten Musterelements an dieser Seite bildet.

Nach der bevorzugten Ausgestaltung gemäß Anspruch 6 weisen einzelne oder alle Musterelemente jeweils eine punktsymmetrische und/oder eine achsensymmetrische Außenkontur auf. Einzelne oder alle Musterelemente können auch eine runde, insbesondere kreis- oder ovalförmige, und/oder eckige, insbesondere rechteckige oder rautenförmige, vorzugsweise hexagonale, Außenkontur aufweisen. Es ist auch denkbar, dass Musterelemente mehreckig mit gebogenen Kanten ausgestaltet sind. Besonders bevorzugt weist das Bondingmuster insgesamt eine Hexagonalstruktur auf. Durch Musterelemente in Form von Hexagonen, die insbesondere ohne Zwischenräume zu einer Hexagonalstruktur angeordnet sind, ist die von den Musterelementen eingenommene Fläche der Vliesschicht besonders gleichmäßig und vollständig gebondet bzw. verfestigt.

Anspruch 7 definiert bevorzugte Ausgestaltungen der Außenkontur der Musterelemente mittels Prägeelementen, die beim Bonden erzeugt werden. Bei den Prägeelementen handelt es sich insbesondere um die die Außenkontur bildenden Prägepunkte und/oder mindestens eine, insbesondere genau eine, Prägelinie, die die Außenkontur bildet. Grundsätzlich können auch Kombinationen von Prägepunkten und Prägelinien vorgesehen sein, um eine Außenkontur eines Musterelements zu bilden. Einzelne oder alle Prägepunkte können eine punktsymmetrische und/oder achsensymmetrische Querschnittsform aufweisen. Zusätzlich oder alternativ können einzelne oder alle Prägepunkte eine runde, insbesondere kreis- oder ovalförmige, eine eckige, insbesondere rechteckige oder rautenförmige, und/oder eine streifenförmige Querschnittsform aufweisen. Im Bereich dieser Prägeelemente hat dann die Vliesschicht ihre geringste Dicke.

Besonders bevorzugt ist die Vliesschicht nach der weiteren Ausgestaltung gemäß Anspruch 8 auf einem Vliesschicht-Trägermaterial, insbesondere einer Folie, aufgebracht. Das Vliesschicht-Trägermaterial bildet zusammen mit der Vliesschicht dann vorzugsweise das gesamte Rückenblatt.

Die Ansprüche 9 und 10 definieren besonders bevorzugte Längenverhältnisse und Dickenverhältnisse innerhalb eines jeweiligen Musterelements. Diese Längenverhältnisse bzw. Dickenverhältnisse haben sich als besonders geeignet für die vorschlagsgemäße Windel herausgestellt.

Die Ansprüche 11 bis 13 definieren besonders bevorzugte Eigenschaften der Fasern, die die Vliesschicht des Rückenblatts bilden.

Nach der besonders bevorzugten Ausgestaltung gemäß Anspruchs 14 sind im Haltezustand eine oder mehrere Fasern, wie dies bereits zuvor erläutert wurde, zumindest an zwei Stellen fixiert, jedenfalls an mindestens einer ersten Verbindungsstelle im Bereich des Prägeelements und/oder am Vliesschicht-Trägermaterial einerseits und an mindestens einer zweiten Verbindungsstelle am Kleberbereich der Befestigungslasche andererseits. Diese zumindest zweifache und gegebenenfalls auch mehrfache Fixierung von Fasern erhöht deren Vermögen Haltekräfte aufzunehmen deutlich. Mindestens ein Haken des Hakenbereichs, vorzugsweise eine Mehrzahl von Haken, kann dann mit einem zwischen den Verbindungsstellen verlaufenden Faserabschnitt verhakt sein (Anspruch 15). Dadurch werden Bewegungen des jeweils verhakten Hakens entlang der Fasern, mit denen er verhakt ist, und insbesondere auch quer dazu begrenzt. Grundsätzlich können dabei auch weitere Fasern nur über eine der beiden genannten Verbindungsstellen und/oder nur über die an den beiden Verbindungsstellen fixierten Fasern fixiert sein. Insbesondere können auch weitere Haken vorgesehen sein, die nur mit den weiteren Fasern, die also nicht an beiden Verbindungsstellen fixiert sind, verhakt sein.

Im Folgenden wird die Erfindung anhand einer lediglich ein Ausführungsbeispiel darstellenden Zeichnung näher erläutert. In der Zeichnung zeigt
- Fig. 1: in einer schematischen Perspektivansicht eine vorschlagsgemäße wegwerfbare Windel,
- Fig. 2: in einer Draufsicht mit übereinstimmenden Größenverhältnissen in a) einen Ausschnitt eines Bondingmusters der Vliesschicht der Windel gemäß Fig. 1 und in b) einen Ausschnitt einer Befestigungslasche einer Windel gemäß Fig. 1,
- Fig. 3: in einer übereinandergelegten Ansicht die Vliesschicht mit dem Bondingmuster und die Befestigungslasche gemäß Fig. 2 und
- Fig. 4: eine schematische Schnittansicht im Bereich eines Musterelements im Haltezustand bei zusammengeführter Befestigungslasche und Rückenblatt.

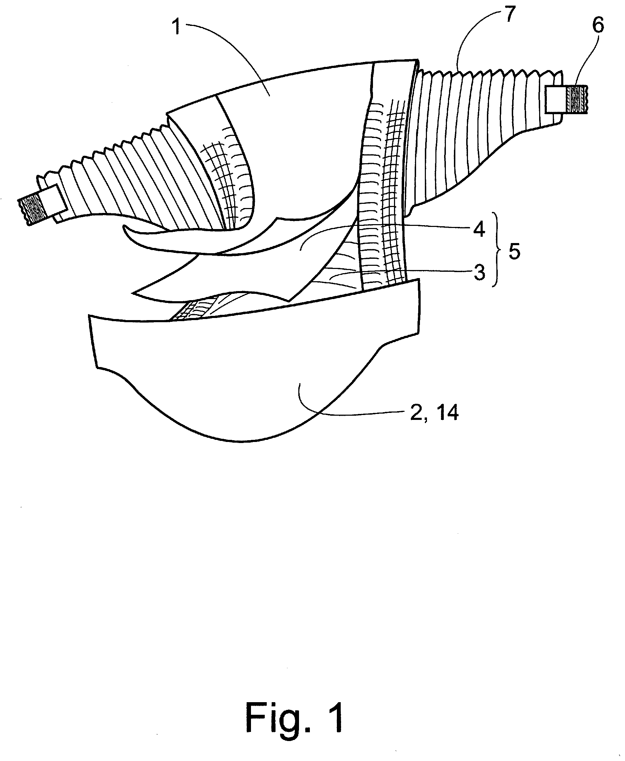

In der perspektivischen Ansicht gemäß Fig. 1 ist beispielhaft eine wegwerfbare Windel, hier in Form einer Babywindel, dargestellt. Grundsätzlich kann es sich bei der Windel aber auch um eine Erwachsenenwindel oder Inkontinenzwindeln handeln. Die vorschlagsgemäße Windel weist ein hier und vorzugsweise flüssigkeitsdurchlässiges, körperseitiges Deckblatt 1, auch Topsheet genannt, und ein hier und vorzugsweise flüssigkeitsundurchlässiges, körperfernes Rückenblatt 2, auch Backsheet genannt, auf. Zwischen dem Deckblatt 1 und dem Rückenblatt 2 sind weitere Lagen in dem Schichtaufbau vorgesehen, nämlich ein Saugkörper 3 und eine Verteilerlage 4, die zusammen eine flüssigkeitsabsorbierende Sauganordnung 5 bilden. Ferner weist die Windel mindestens eine Befestigungslasche 6, hier genau zwei Befestigungslaschen 6, auf, die an Seitenflügeln 7 der Windel angeordnet sind. Die Seitenflügel 7 sind mit dem Schichtaufbau der Windel, insbesondere dem Rückenblatt 2, verbunden, insbesondere verklebt, oder einstückig damit ausgeführt. Die jeweilige Befestigungslasche 6 ist insbesondere mit dem zugeordneten Seitenflügel 7 verbunden, insbesondere verklebt, oder einstückig damit ausgeführt.

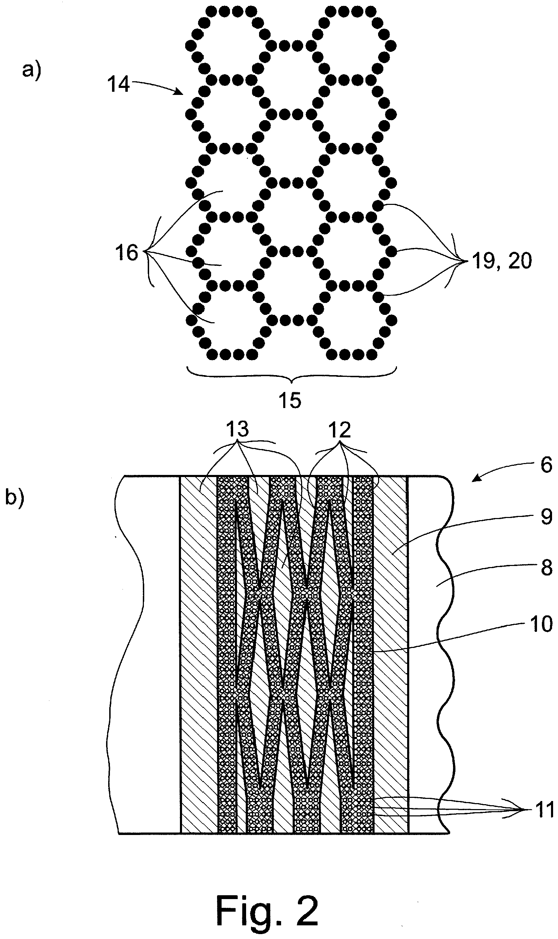

Die jeweilige Befestigungslasche 6 weist, wie Fig. 2b) veranschaulicht, hier und vorzugsweise mehrere Lagen auf, von denen eine ein Trägermaterial 8, insbesondere in Form einer Folie, bildet, wobei auf dem Trägermaterial 8 eine hier und vorzugsweise durchgehende Kleberschicht 9 und darauf wiederrum ein Hakenband 10 mit einer Vielzahl von einzelnen Haken 11 angeordnet ist. Die Kleberschicht 9 verbindet somit das Hakenband 10 stoffschlüssig mit dem Trägermaterial 8. Dabei ist das Hakenband 10 in seiner Längsrichtung mehrfach geschlitzt und bildet somit im auseinander gezogenen Zustand ein Streckgitter. Das Streckgitter besteht aus einer Vielzahl von einzelnen mit Haken 11 versehenen Stegen, die sogenannte Hakenbereiche 12 definieren. In den Zwischenräumen des Streckgitters, das aus dem Hakenband 10 erzeugt worden ist, tritt Kleber der Kleberschicht 9 zum Vorschein, wobei diese Bereiche als Kleberbereiche 13 bezeichnet werden. Außerdem befindet sich zu jeder Seite des Hakenbands 10 je ein weiterer streifenförmiger Kleberbereich 13.

Eine solche Befestigungslasche 6 wird zur bestimmungsgemäßen Befestigung der Windel am Körper des Benutzers mit dem Rückenblatt 2, und zwar insbesondere dessen in Fig. 1 nach vorne gerichtetem Teil des Rückenblatts 2, der dann als Komfortbündchen fungiert, zusammengeführt. Zur Verbindung mit der Befestigungslasche 6 weist das Rückenblatt 2 an seiner vom Deckblatt 1 abgewandten Seite (Windelaußenseite) eine Vliesschicht 14 auf, die sich hier und vorzugsweise zumindest über den größten Teil der Fläche des Rückenblatts 2, hier insbesondere die gesamte Fläche des Rückenblatts 2, erstreckt. Die Befestigungslasche 6 ist somit an jeder Stelle des Rückenblatts 2 mit diesem in Kontakt bringbar.

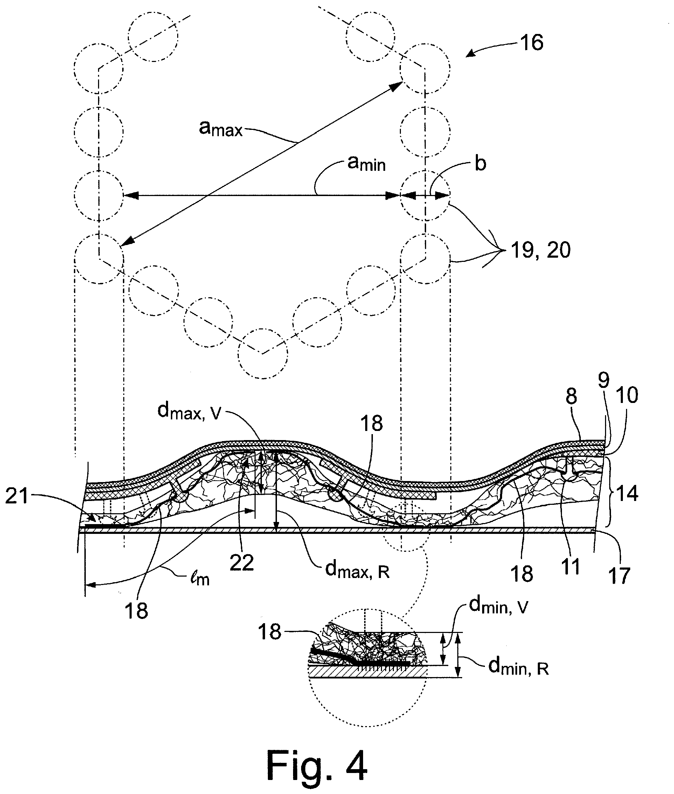

Die Vliesschicht 14, auch Nonwoven-Schicht genannt, ist vorschlagsgemäß in einem Bondingmuster 15 aus Musterelementen 16 gebondet, also verfestigt. Hier und vorzugsweise ist die Vliesschicht 14 zusammen mit einem Vliesschicht-Trägermaterial 17, das beispielsweise in Form einer Folie vorliegt, gebondet, hier und vorzugsweise punktförmig gebondet, was beispielsweise in einem Kalander unter erhöhtem Druck und erhöhter Temperatur durchgeführt worden ist. Durch das Bonden werden die die Vliesschicht 14 bildenden Fasern 18 komprimiert und zumindest miteinander und gegebenenfalls auch mit dem Vliesschicht-Trägermaterial 17 verschmolzen bzw. verschweißt, wodurch Konturen in der Vliesschicht 14 erzeugt werden, die die Musterelemente 16 bilden, die zusammen wiederrum das Bondingmuster 15 bilden. An den Stellen, an denen die Vliesschicht 14 gebondet worden ist, entstehen sogenannte Prägeelemente 19, bei dem hier punktförmigen Bonden Prägepunkte 20, hier beispielhaft mit kreisförmiger Querschnittsform. Fig. 2a) zeigt eine Vielzahl solcher Prägepunkte 20, von denen mehrere die Außenkontur der jeweiligen Musterelemente 16 bilden. Im übrigen Bereich innerhalb dieser Außenkontur weist die Vliesschicht 14 ein größeres Volumen als an den Prägeelementen 19 bzw. Prägepunkten 20 auf. Dies ist beispielsweise auch in Fig. 4 zu erkennen, die im Weiteren noch näher erläutert wird.

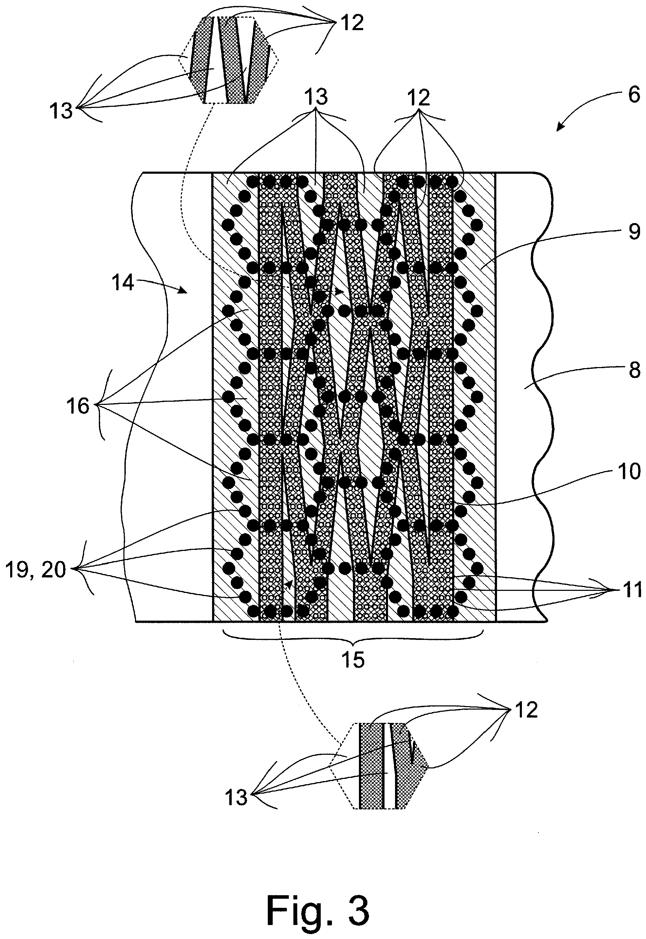

Im Haltezustand, wenn nun also die jeweilige Befestigungslasche 6 mit der Vliesschicht 14 zusammengeführt ist, kommen nun, wie Fig. 3 veranschaulicht, die Hakenbereiche 12 und die Kleberbereiche 13 jeweils mit der Vliesschicht 14, die mit dem Bondingmuster 15 versehen ist, in Kontakt. In der in Fig. 3 dargestellten, schematischen Draufsicht, die tatsächliche Größenverhältnisse darstellt, ist zu erkennen, dass innerhalb eines jeden Musterelements 16 die Hakenbereiche 12 einerseits und die Kleberbereiche 13 andererseits jeweils einen bestimmten Flächenanteil bezogen auf die Gesamtfläche des jeweiligen Musterelements 16 einnehmen. Dabei sind die Hakenbereiche 12, die Kleberbereiche 13 und die Musterelemente 16 so geformt bzw. weisen solche Abmessungen auf, dass die Kleberbereiche 13 im Haltezustand einen mittleren Flächenanteil von mindestens 20% bezogen auf die Gesamtfläche des jeweiligen Musterelements 16 einnehmen. Vorzugsweise nehmen die Kleberbereiche 13 einen mittleren Flächenanteil von mindestens 30%, vorzugsweise von mindestens 35%, weiter vorzugsweise von mindestens 40%, ein. Zusätzlich oder alternativ kann vorgesehen sein, dass die Kleberbereiche 13 einen mittleren Flächenanteil von höchstens 60%, vorzugweise höchstens 55%, weiter vorzugsweise höchstens 50%, einnehmen. In dem hier dargestellten und insoweit bevorzugten Ausführungsbeispiel nehmen die Kleberbereiche 13 einen mittleren Flächenanteil von 45% ein.

Die Hakenbereiche 12 wiederrum können einen mittleren Flächenanteil von mindestens 55% bezogen auf die Gesamtfläche des jeweiligen Musterelements 16 einnehmen, vorzugsweise einen mittleren Flächenanteil von mindestens 60%, weiter vorzugsweise mindestens 65%. Zusätzlich oder alternativ können die Hakenbereiche 12 einen mittleren Flächenanteil von höchstens 50%, vorzugsweise höchstens 45% und weiter vorzugsweise höchstens 40% einnehmen. In dem hier dargestellten und insoweit bevorzugten Ausführungsbeispiel nehmen die Hakenbereiche 12 einen mittleren Flächenanteil von 55% ein.

Durch die zuvor beschriebenen bevorzugten Wertebereiche für die mittleren Flächenanteile ist gewährleistet, dass eine optimale Fläche an Kleber zur Verfügung steht, an der die Fasern 18 der Vliesschicht 14 im Haltezustand anhaften können. Auf diese Weise wird ein optimaler Anteil an Fasern 18 zum einen im Bereich der Prägeelemente 19 und zum anderen im Bereich des Kontakts mit dem Kleber der Kleberbereiche 13 fixiert. Die so an zumindest zwei Verbindungsstellen fixierten Fasern 18 haben einen besonders guten Halt und können entsprechend große Haltekräfte aufnehmen, wenn einzelne Haken 11 der Hakenbereiche 12 mit diesen Fasern 18 verhakt sind und Zugbelastungen orthogonal zur Verlaufsebene des Rückenblatts 2 oder entlang dieser von den Haken 11 auf die Fasern 18 ausgeübt werden.

Die Musterelemente 16, die zusammen das Bondingmuster 15 bilden, nehmen, in vertikaler Projektion, hier und vorzugsweise zumindest den größten Teil der Fläche der Vliesschicht 14, hier die gesamte Fläche der Vliesschicht 14, ein. Auf diese Weise ist über die gesamte Erstreckung der Vliesschicht 14 und vorzugsweise auch über die gesamte Erstreckung des Rückenblatts 2 eine gleichermaßen optimale Befestigung der jeweiligen Befestigungslasche 6 an der Vliesschicht 14 bzw. dem Rückenblatts 2 gewährleistet.

Um die Befestigungsqualität weiter zu optimieren, sind die Musterelemente 16 hier und vorzugsweise ohne Zwischenräume angeordnet und/oder weisen auch alle denselben Querschnitt, das heißt dieselbe Querschnittsform und -fläche, auf, was Fig. 2a) veranschaulicht. Zumindest einzelne, hier alle Musterelemente 16 weisen zudem hier und vorzugsweise jeweils eine punktsymmetrische und/oder achsensymmetrische Außenkontur auf. Einzelne oder alle Musterelemente 16 können dabei eine runde, insbesondere kreis- oder ovalförmige, und/oder eine eckige, insbesondere rechteckige oder rautenförmige, Außenkontur aufweisen. Es ist auch denkbar, dass Musterelemente 16 mehreckig mit gebogenen Kanten ausgestaltet sind. Im vorliegenden Ausführungsbeispiel haben alle Musterelemente 16 eine hexagonale Außenkontur, wodurch das Bondingmuster 15 eine Hexagonalstruktur aufweist.

Wie bereits zuvor erläutert, wird die Außenkontur der Musterelemente 16 von einem oder mehreren Prägeelementen 19, hier und vorzugsweise einer Vielzahl von zueinander insbesondere gleichmäßig beabstandeten Prägepunkten 20, gebildet, die sich entlang des Umfangs des jeweiligen Musterelements 16 erstrecken. Im vorliegenden Fall des punktförmigen Bondens, also des Vorliegens einer Vielzahl von Prägepunkten 20, wird die Außenkontur des jeweiligen Musterelements 16 durch eine gedachte Verbindungslinie gebildet, die die Mittelpunkte der zueinander am nächsten liegenden Prägepunkte 20 miteinander verbindet. Da die Musterelemente 16 hier ohne Zwischenräume angeordnet sind, bildet ein Abschnitt, insbesondere ein gerader Abschnitt, der Außenkontur des einen Musterelements 16 auch einen entsprechenden Abschnitt der Außenkontur des jeweils benachbarten Musterelements 16. Im Bereich des jeweiligen Prägeelements 19 bzw. Prägepunkts 20, im Falle einer Prägelinie auch im Bereich der jeweiligen Prägelinie, weist die Vliesschicht 14 ihre geringste Dicke d_{min,V} auf.

Innerhalb eines Musterelements 16 des Bondingmusters 15 liegen bei der vorschlagsgemäßen Windel besondere Längen- und Dickenverhältnisse zugrunde. Diese sind in Fig. 4 schematisch dargestellt.

So ist hier und vorzugsweise zum einen vorgesehen, dass bei den Musterelementen 16 jeweils das Verhältnis des maximalen Abstands aₘₐₓ zweier einander diametral gegenüberliegender Prägeelemente 19 zu der Breite b der Prägeelemente 19 zwischen 4 und 9, vorzugsweise zwischen 5 und 8, weiter vorzugsweise zwischen 6 und 7, liegt. Zusätzlich oder alternativ kann vorgesehen sein, dass das Verhältnis des minimalen Abstands aₘᵢₙ zweier einander diametral gegenüberliegender Prägeelemente 19 zu der Breite b der Prägeelemente 19 zwischen 3 und 8, vorzugsweise zwischen 4 und 7, weiter vorzugsweise zwischen 5 und 6, liegt.

Mit einander "diametral" gegenüberliegenden Prägeelementen 19 sind die beiden Prägeelemente 19 gemeint, die auf einer gemeinsamen, durch den Mittelpunkt des jeweiligen Musterelements 16 verlaufenden, gedachten Linie liegen. Bei einem kreisförmigen Musterelement 16 ist diese Linie der Durchmesser, bei einem nicht-kreisförmigen, hier beispielsweise hexagonalen Musterelement ist diese Linie eine durch den Mittelpunkt verlaufende Diagonale. Insoweit ist der Begriff "diametral" nicht nur auf kreisförmige Außenkonturen, sondern auf jedwede Form von Außenkonturen, insbesondere auch hexagonale Außenkonturen bezogen und entsprechend weit zu verstehen.

Weiterhin ist hier und vorzugsweise vorgesehen, dass bei den Musterelementen 16 jeweils das Verhältnis der maximalen Dicke d_{max,V} der Vliesschicht 14 innerhalb des jeweiligen Musterelements 16 zu der minimalen Dicke d_{min,V}. d_{min,R} der Vliesschicht 14 oder des Rückenblatts 2 im Bereich der Prägeelemente 19 zwischen 2 und 7, vorzugsweise zwischen 3 und 6, weiter vorzugsweise zwischen 4 und 5, liegt. Zusätzlich oder alternativ kann vorgesehen sein, dass das Verhältnis der maximalen Dicke d_{max,R} des Rückenblatts 2 innerhalb des Bereichs des jeweiligen Musterelements 16 zu der minimalen Dicke d_{min,V}, d_{min,R} der Vliesschicht 14 oder des Rückenblatts 2 im Bereich der Prägeelemente 19 zwischen 5 und 10, vorzugsweise zwischen 6 und 9, weiter vorzugsweise zwischen 7 und 8, liegt.

Auch eine spezielle mittlere Faserlänge der die Vliesschicht 14 bildenden Fasern 18 kann bei der vorschlagsgemäßen Windel vorteilhaft sein. So beträgt hier und vorzugsweise die mittlere Faserlänge der Fasern 18 mindestens ein Drittel, vorzugsweise mindestens die Hälfte, des minimalen Abstands aₘᵢₙ oder maximalen Abstands aₘₐₓ zweier einander diametral gegenüberliegender Prägeelemente 19 bzw. Prägepunkte 20. Vorzugsweise entspricht die mittlere Faserlänge der Fasern 18 sogar mindestens dem minimalen Abstand aₘᵢₙ oder maximalen Abstand aₘₐₓ der zwei einander diametral gegenüberliegenden Prägeelemente 19 bzw. Prägepunkte 20 und kann auch ein Mehrfaches des minimalen Abstands aₘᵢₙ oder maximalen Abstands aₘₐₓ der zwei einander diametral gegenüberliegenden Prägeelemente 19 bzw. Prägepunkte 20 betragen. Die mittlere Faserlänge Iₘ kann für die vorschlagsgemäße Windel zwischen 3 und 130 mm liegen. Vorzugsweise liegt die mittlere Faserlänge Iₘ zwischen 25 und 130 mm, weiter vorzugsweise zwischen 40 und 80 mm, was insbesondere für Fasern 18 eines Spinnvlieses gilt. Im Falle eines Spinnvlieses kann es sich bei den Fasern 18 auch um Endlosfasern handeln. Insbesondere bei Stapelfasern kann die mittlere Faserlänge Iₘ auch zwischen 3 und 30 mm, weiter vorzugsweise zwischen 5 und 20 mm, liegen.

Weiterhin ist es vorteilhaft, wenn die Faserfeinheit der Fasern 18 zwischen 0,05 und 2,5 dtex, vorzugsweise zwischen 0,5 und 2,5 dtex, weiter vorzugsweise zwischen 1,5 und 2,5 dtex, liegt. Hier handelt es sich bei der Vliesschicht 14 beispielhaft um ein Spinnvlies aus Fasern 18 mit einer Faserfeinheit von 1,7 dtex. Zusätzlich oder alternativ kann die Faserstoffdichte der Vliesschicht 14 zwischen 10 und 20 g/m², vorzugsweise zwischen 12 und 18 g/m², weiter vorzugsweise zwischen 13 und 17 g/m², liegen. Mit der Faserfeinheit ist dabei insbesondere die mittlere Faserfeinheit der die Vliesschicht bildenden Fasern 18 gemeint. Mit der Faserstoffdichte ist entsprechend insbesondere die mittlere Faserstoffdichte der Vliesschicht 14 gemeint.

Fig. 4 veranschaulicht, was bereits zuvor erläutert wurde, dass im Haltezustand hier und vorzugsweise eine oder mehrere der Fasern 18 an mindestens einer ersten Verbindungsstelle im Bereich des Prägeelements 19 bzw. Prägepunkts 20 und/oder am Vliesschicht-Trägermaterial 17 einerseits und an mindestens einer zweiten Verbindungsstelle 22 am Kleberbereich 13 der Befestigungslasche 6 andererseits fixiert, insbesondere stoffschlüssig fixiert, sind. Greift nun ein Haken 11 der Hakenbereiche 12 unter eine solche an den mindestens zwei Verbindungsstellen 21, 22 fixierte Faser 18, was in Fig. 4 anhand beispielhaft hervorgehobener Fasern 18 gezeigt ist, begrenzt die Faser 18 den Bewegungsspielraum des jeweiligen Hakens 11, und zwar nicht nur in Richtung orthogonal zu der Verlaufsebene des Rückenblatts 2, sondern insbesondere auch in Richtung entlang der Verlaufsebene. Im Haltezustand ist also hier und vorzugsweise mindestens ein Haken 11 bzw. sind mehrere Haken 11 jeweils mit einem zwischen den Verbindungsstellen 21, 22 verlaufenden Faserabschnitt der jeweiligen Faser bzw. Fasern 18, die jeweils an der ersten und zweiten Verbindungsstelle 21, 22 fixiert ist bzw. sind, verhakt. Auf diese Weise wird, insbesondere mit den zuvor beschriebenen Längen- und/oder Dickenverhältnissen, eine besonders sichere Verbindung zwischen der jeweiligen Befestigungslasche 6 und der Vliesschicht 14 bzw. dem Rückenblatt 2 im Haltezustand gewährleistet.

## Patentansprüche

1. Wegwerfbare Windel, insbesondere Babywindel, mit einem körperseitigen Deckblatt (1), mit einem körperfernen Rückenblatt (2) und mit mindestens einer Befestigungslasche (6), wobei das Rückenblatt (2) eine Vliesschicht (14) aufweist und wobei die Befestigungslasche (6) Hakenbereiche (12) und Kleberbereiche (13) aufweist, die im Haltezustand jeweils mit dem Rückenblatt (2) in Kontakt kommen,
**dadurch gekennzeichnet,**
**dass** die Vliesschicht (14) des Rückenblatts (2) in einem Bondingmuster (15) aus Musterelementen (16) gebondet ist und dass im Haltezustand innerhalb der Musterelemente (16) die Kleberbereiche (13) einen mittleren Flächenanteil von mindestens 20% bezogen auf die Gesamtfläche des jeweiligen Musterelements (16) einnehmen.

2. Wegwerfbare Windel nach Anspruch 1, **dadurch gekennzeichnet, dass** im Haltezustand innerhalb der Musterelemente (16) die Kleberbereiche (13) einen mittleren Flächenanteil von mindestens 30%, vorzugsweise mindestens 35%, weiter vorzugsweise mindestens 40%, weiter vorzugsweise 45%, und/oder einen mittleren Flächenanteil von höchstens 60%, vorzugsweise höchstens 55%, weiter vorzugsweise höchstens 50%, bezogen auf die Gesamtfläche des jeweiligen Musterelements (16) einnehmen.

3. Wegwerfbare Windel nach Anspruch 1 oder 2, **dadurch gekennzeichnet, dass** im Haltezustand innerhalb der Musterelemente (16) die Hakenbereiche (12) einen mittleren Flächenanteil von mindestens 55%, vorzugsweise mindestens 60%, weiter vorzugsweise mindestens 65%, und/oder einen Flächenanteil von höchstens 50%, vorzugsweise höchstens 45%, weiter vorzugsweise höchstens 40%, bezogen auf die Gesamtfläche des jeweiligen Musterelements (16) einnehmen.

4. Wegwerfbare Windel nach einem der vorhergehenden Ansprüche, **dadurch gekennzeichnet, dass** die Musterelemente (16) zumindest den größten Teil der Fläche der Vliesschicht (14), vorzugsweise die gesamte Fläche der Vliesschicht (14), einnehmen, und/oder, dass die Vliesschicht (14) zumindest den größten Teil der Fläche des Rückenblatts (2), insbesondere die gesamte Fläche des Rückenblattes (2), einnimmt.

5. Wegwerfbare Windel nach einem der vorhergehenden Ansprüche, **dadurch gekennzeichnet, dass** die Musterelemente (16) ohne Zwischenräume angeordnet sind und/oder alle Musterelemente (16) denselben Querschnitt aufweisen.

6. Wegwerfbare Windel nach einem der vorhergehenden Ansprüche, **dadurch gekennzeichnet, dass** einzelne oder alle Musterelemente (16) jeweils eine punktsymmetrische und/oder achsensymmetrische Außenkontur aufweisen, und/oder, dass einzelne oder alle Musterelemente (16) jeweils eine runde und/oder eckige, vorzugsweise hexagonale, Außenkontur aufweisen, vorzugsweise, dass das Bondingmuster (15) eine Hexagonalstruktur aufweist.

7. Wegwerfbare Windel nach einem der vorhergehenden Ansprüche, **dadurch gekennzeichnet, dass** die Außenkontur der Musterelemente (16) von einem oder mehreren Prägeelementen (19), insbesondere einer Vielzahl von zueinander, insbesondere gleichmäßig, beabstandeten Prägepunkten (20) und/oder mindestens einer, insbesondere genau einer, Prägelinie, gebildet wird, die entlang des Umfangs des jeweiligen Musterelements (16) angeordnet sind, wobei im Bereich des jeweiligen Prägeelements (19) die Vliesschicht (14) ihre minimale Dicke (d_{min,V}) aufweist, vorzugsweise, dass einzelne oder alle Prägepunkte (20) jeweils eine punktsymmetrische und/oder achsensymmetrische Querschnittsform aufweisen, und/oder, dass einzelne oder alle Prägepunkte (20) jeweils eine runde, eckige und/oder streifenförmige Querschnittsform aufweisen.

8. Wegwerfbare Windel nach einem der vorhergehenden Ansprüche, **dadurch gekennzeichnet, dass** die Vliesschicht (14) auf einem Vliesschicht-Trägermaterial (17), insbesondere einer Folie, aufgebracht ist.

9. Wegwerfbare Windel nach einem der vorhergehenden Ansprüche, **dadurch gekennzeichnet, dass** bei den Musterelementen (16) jeweils
- das Verhältnis des maximalen Abstands (aₘₐₓ) zweier einander diametral gegenüberliegender Prägeelemente (19) zu der Breite (b) der Prägeelemente (19) zwischen 4 und 9, vorzugsweise zwischen 5 und 8, weiter vorzugsweise zwischen 6 und 7, liegt und/oder
- das Verhältnis des minimalen Abstands (aₘᵢₙ) zweier einander diametral gegenüberliegender Prägeelemente (19) zu der Breite (b) der Prägeelemente (19) zwischen 3 und 8, vorzugsweise zwischen 4 und 7, weiter vorzugsweise zwischen 5 und 6, liegt.

10. Wegwerfbare Windel nach einem der vorhergehenden Ansprüche, **dadurch gekennzeichnet, dass** bei den Musterelementen (16) jeweils
- das Verhältnis der maximalen Dicke (d_{max,V}) der Vliesschicht (14) innerhalb des jeweiligen Musterelements (16) zu der minimalen Dicke (d_{min,V}, d_{min,R}) der Vliesschicht (14) oder des Rückenblatts (2) im Bereich der Prägeelemente (19) zwischen 2 und 7, vorzugsweise zwischen 3 und 6, weiter vorzugsweise zwischen 4 und 5, liegt und/oder
- das Verhältnis der maximalen Dicke (d_{max,R}) des Rückenblatts (2) innerhalb des Bereichs des jeweiligen Musterelements (16) zu der minimalen Dicke (d_{min,V}, d_{min,R}) der Vliesschicht (14) oder des Rückenblatts (2) im Bereich der Prägeelemente (19) zwischen 5 und 10, vorzugsweise zwischen 6 und 9, weiter vorzugsweise zwischen 7 und 8, liegt.

11. Wegwerfbare Windel nach einem der vorhergehenden Ansprüche, **dadurch gekennzeichnet, dass** die mittlere Faserlänge (Iₘ) der die Vliesschicht (14) bildenden Fasern (18) mindestens ein Drittel, vorzugsweise mindestens die Hälfte, des minimalen Abstands (aₘᵢₙ) oder maximalen Abstands (aₘₐₓ) zweier einander diametral gegenüberliegender Prägeelemente (19) beträgt, vorzugsweise, dass die mittlere Faserlänge (Iₘ) mindestens dem minimalen Abstand (aₘᵢₙ) oder maximalen Abstand (aₘₐₓ) zweier einander diametral gegenüberliegender Prägeelemente (19) entspricht.

12. Wegwerfbare Windel nach einem der vorhergehenden Ansprüche, **dadurch gekennzeichnet, dass** die mittlere Faserlänge (Iₘ) der die Vliesschicht (14) bildenden Fasern (18) zwischen 3 und 130 mm liegt, vorzugsweise, dass die mittlere Faserlänge (Iₘ) der die Vliesschicht (14) bildenden Fasern (18) zwischen 30 und 130 mm, weiter vorzugsweise zwischen 40 und 80 mm, oder dass die mittlere Faserlänge (Iₘ) der die Vliesschicht (14) bildenden Fasern (18) zwischen 3 und 30 mm, weiter vorzugsweise zwischen 5 und 20 mm, liegt.

13. Wegwerfbare Windel nach einem der vorhergehenden Ansprüche, **dadurch gekennzeichnet, dass** die Faserfeinheit der die Vliesschicht (14) bildenden Fasern (18) zwischen 0,05 und 2,5 dtex, vorzugsweise zwischen 0,5 und 2,5 dtex, weiter vorzugsweise zwischen 1,5 und 2,5 dtex, liegt, und/oder, dass die Faserstoffdichte der Vliesschicht (14) zwischen 10 und 20 g/m², vorzugsweise zwischen 12 und 18 g/m², weiter vorzugsweise zwischen 13 und 17 g/m², liegt.

14. Wegwerfbare Windel nach einem der vorhergehenden Ansprüche, **dadurch gekennzeichnet, dass** im Haltezustand eine oder mehrere der die Vliesschicht (14) bildenden Fasern (18) an mindestens einer ersten Verbindungsstelle (21) im Bereich des jeweiligen Prägeelements (19) und/oder am Vliesschicht-Trägermaterial (17) und an mindestens einer zweiten Verbindungsstelle (22) am Kleberbereich (13) der Befestigungslasche (6) fixiert, insbesondere stoffschlüssig fixiert, sind.

15. Wegwerfbare Windel nach Anspruch 14, **dadurch gekennzeichnet, dass** im Haltezustand der Hakenbereich (12) der Befestigungslasche (6) eine Vielzahl von Haken (11) aufweist, von denen mindestens ein Haken (11) jeweils mit einem zwischen den Verbindungsstellen (21, 22) verlaufenden Faserabschnitt der einen oder mehreren Fasern (18), die jeweils an der ersten und zweiten Verbindungsstelle (21, 22) fixiert sind, verhakt ist.
